# EUROPÄISCHE PATENTANMELDUNG

(11) **EP 2 058 387 A2**
(43) Veröffentlichungstag der Anmeldung: **13.05.2009**
(21) Anmeldenummer: 08019487.1
(22) Anmeldetag: 07.11.2008
(51) Int. Cl.: C12M 1/113, C12P 5/02

(54) **Verfahren und Vorrichtung zur Energiegewinnung in Biogasanlagen**

(30) Priorität: 08.11.2007 DE 102007053583; 19.11.2007 DE 102007055368; 03.03.2008 DE 102008012182
(71) Anmelder: Schütze, Harald, 01623 Lommatzsch (DE)
(72) Erfinder: Schütze, Harald, 01623 Lommatzsch (DE)
(74) Vertreter: Dinter, Tilo

(57) **Zusammenfassung**

Insbesondere dient die Erfindung zur Aufbereitung von Mischgütern aus Silierstoffen innerhalb von Biogasanlagen. Die Aufgabe der Erfindung besteht in der Schaffung eines Verfahrens und einer Vorrichtung zur optimalen Energiegewinnung durch effiziente Aufbereitung und Zwischenlagerung von Mischgütern zwecks optimaler Energieausbeute der zur Anwendung kommenden pflanzlichen Mischungskomponenten bei geringem technischen Aufwand und zur Reduzierung der erforderlichen Abfackelungsvorgänge. Gelöst wird die Aufgabe indem der Mischvorgang durch technische Regelungen der Zuführungen und Mischungsvorgänge von zerkleinerten, festen pflanzlichen Stoffen mit als Gleitmittel dienenden pflanzlichen Suspensionen erfolgt. Die Vorrichtung zur Durchführung des Verfahrens ist so gestaltet, dass die Zufuhrung der aufbereiteten dickflüssigen Biomasse mittels in unterschiedlichen Höhen innerhalb der Vorrichtung angeordneten Zufuhrkanälen erfolgt, die Bodenfläche (18) der Vorrichtung mindestens eine nach oben hin sich verjüngende Erhebung (19) aufweist, an der Bodenfläche (18) zwischen den Erhebungen (19) mindestens ein Entnahmekanal (20) angeordnet ist und den Oberflächen der Erhebungen (19) eine steuerbare Reinigungsvorrichtung (17) sowie eine Heizeinrichtung funktionell zugeordnet sind.

## Beschreibung

Die Erfindung betrifft ein Verfahren und eine Vorrichtung zur Optimierung der Energiegewinnung in Biogasanlagen, insbesondere zur Aufbereitung von Mischgütern aus Silierstoffen innerhalb von Biogasanlagen. Das Verfahren dient der Erreichung einer optimalen Energieausbeute durch eine effiziente technische Regelung der Mischung und Zwischenlagerung der Silierstoffe. Eine weitere Anwendung der Erfindung besteht in der Nutzung während des Ausfalls (Havarie) der zur Umwandlung der Biogasenergie in Wärmeenergie dienenden Baugruppe.

Bekannt sind Anlagen zur Erzeugung von Biogas, die zur Aufbereitung von Stoffen für Biogasreaktoren eine Mischung von Biomasse mit vergärbaren Suspensionen vornehmen. Dazu kommen Mischvorrichtungen, wie beispielsweise der in der DE 20 2004 018 619 U1 beschriebene Zwangsmischer zur Anwendung, die eine homogene Verteilung und gleichmäßige Viskosität der Mischungskomponenten bewirken. Nachteilig an den bekannten Verfahren und Einrichtungen ist jedoch, dass eine regelbare optimale Energieausbeute der verwendeten Mischgütern nicht möglich ist. Bekannt sind weiterhin Verfahren zum Abfackeln von Biogasen wie beispielsweise das Verfahren nach der DD 301 339 A7, bei dem der Gasstrom während des Abfackelvorganges solchen Bedingungen unterworfen wird, dass er mit einer ohne Fremdgas gespeisten Zündflamme sicher entzündet und vollständig verbrannt wird. Nachteilig ist an diesem Verfahren jedoch, dass die Biogasenergie durch den Abfackelungsvorgang ungenutzt in die Atmosphäre abgegeben wird und zur Umweltbelastung beiträgt.

Bekannte Vorrichtungen zur Durchführung des Verfahrens fordern aufgrund der funktionellen Abläufe entsprechende Aufnahmebehälter mit äußeren Abmessungen, die keine Anpassungen an standardisierte Containerabmessungen ermöglichen und dadurch keine Optimicrungen von Lagerkapazitäten sowie logistischen Abläufen gestatten.

Die Aufgabe der Erfindung besteht deshalb in der Schaffung eines Verfahrens zur optimalen Energiegewinnung durch eine effiziente Aufbereitung und Zwischenlagerung von Mischgütern, das eine optimale Energieausbeute der zur Anwendung kommenden pflanzlichen Mischungskomponenten bei einem geringen technischen Aufwand ermöglicht und zur Reduzierung der erforderlichen Abfackelungsvorgänge bei der Biogasherstellung dient. Weiterhin besteht die Aufgabe der Erfindung in der Schaffung einer Vorrichtung zur Durchführung des Verfahrens. Durch die jeweilige Vorgabe von experimentell und messtechnisch ermittelten Mischungsverhältnissen von zerkleinerten festen pflanzlichen Bestandteilen mit pflanzlichen Suspensionen sind die zu regelnden Mengengrößen vorgegeben, die zu einer optimalen Energieausbeute der Biogasanlage führen.

Nachfolgend sollen das Verfahren und die zur Durchführung des Verfahrens dienende Vorrichtung an Hand eines Ausführungsbeispiels näher erläutert werden.

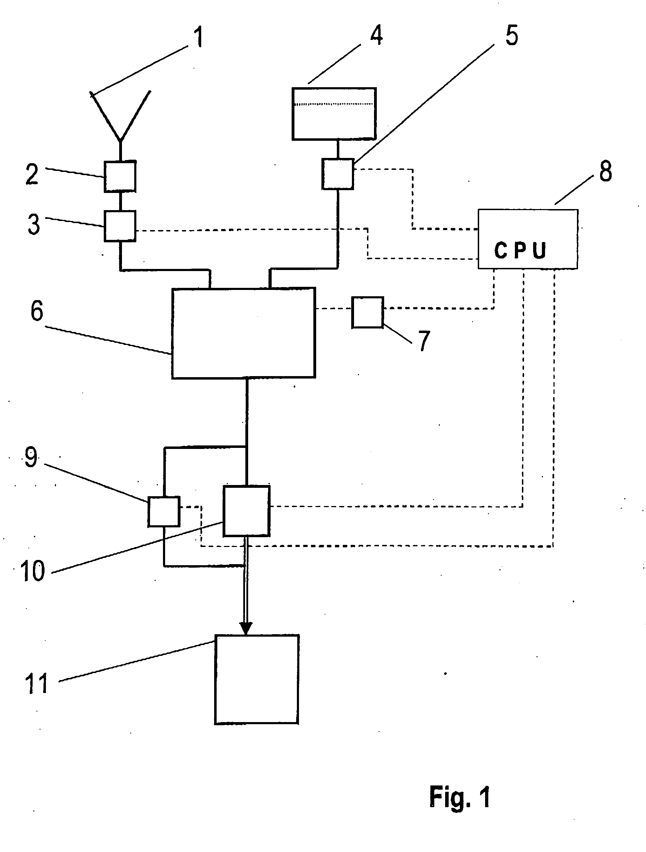

In der Zeichnung zeigt
**Fig. 1****:** den schematischen Ablauf des Verfahrens.
**Fig. 2****:** die schematische Gesamtansicht der Vorrichtung,
**Fig. 3**: die schematische Anordnung kegelförmiger Erhebungen und
**Fig. 4**: die schematische Ansicht pyramidenförmiger Erhebungen.

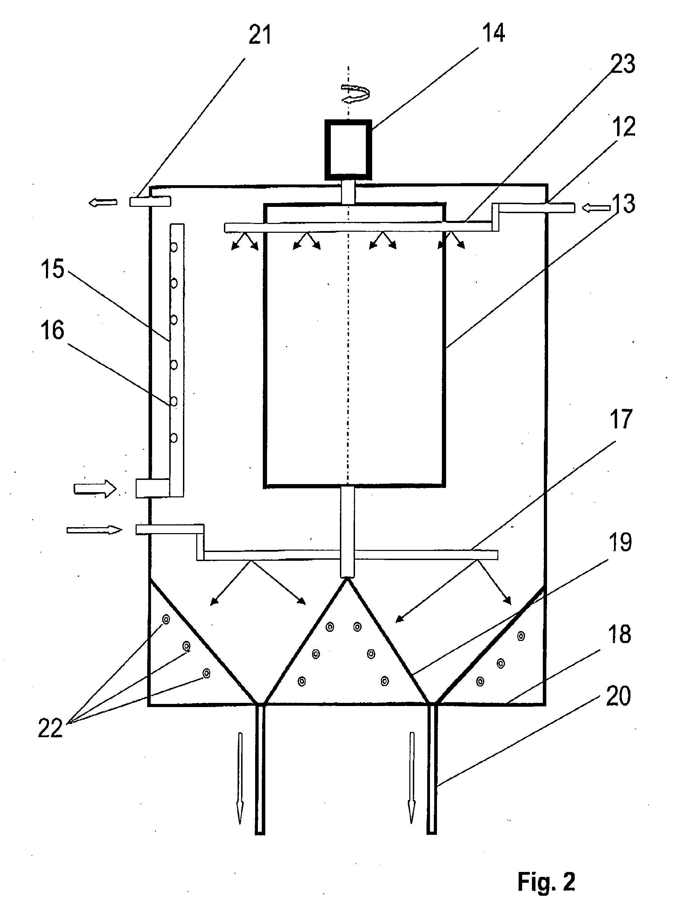

Die Durchführung des Verfahrens zur optimierten Biogaserzeugung setzt eine vorhergehende experimentelle und messtechnische Untersuchung der Mischungsverhältnissc festgelegter Arten bzw. Sorten von festen pflanzlichen Stoffen in Kombination mit vorgegebenen pflanzlichen Suspensionen und den damit erzielten Energieausbeuten voraus. Bei den festen Bestandteilen kann es sich um Ganzpflanzen, Ganzpflanzensilage (Gräser, Mais, u. ä. ), Getreide und deren gemahlene Folgeprodukte, schnell nachwachsende Sträucher, Blattmassen oder Rüben handeln. Die zur Anwendung kommenden pflanzlichen Suspensionen werden beispielsweise aus Gärrcstprodukten, pflanzlichen Ölen bzw. deren Reststoffen, Obst, Wein, Saft, Fruchtprodukte in ihrer Grundsubstanz oder deren Verarbeitungsprodukte bis hin zu exotischen Früchten wie Kiwis, Apfelsinen und anderen Früchten hergestellt. Auf der Grundlage der Vorgabe des Mischungsverhältnisses entsprechend der zur Anwendung kommenden Mischgüter ergeben sich die zuzuführenden Mengen der einzelnen Mischungskomponenten. Mittels an sich bekannter Zerkleincrungsbaugruppen werden die festen Bestandteile nach ihrer Zuführung zerkleinert. So werden im nachfolgend beschriebenen Ausführungsbeispiel die festen Bestandteile von der Schneideinrichtung 1 zerkleinert und danach in dem Zwischenspeicher 2 gespeichert. Die Begrenzung der Zuführungsmenge bei der Zuführung der zwischengespeicherten festen Stoffe in die Mischungsbaugruppe 6 wird von der Dosiereinrichtung 3 vorgenommen. Diese Dosiereinrichtung 3 wird von einer zentralen Steuereinheit 8 entsprechend der vorgegebenen Mengeneinheiten gesteuert. Als zentrale Steuereinheit 8 kommt dabei eine Computereinrichtung zur Anwendung. In Abhängigkeit von dem Füllstand in der Mischungsbaugruppe 6 wird die Zufuhr erhöht oder gedrosselt. Die Erfassung des Füllstandes wird von einem an sich bekannten Sensor 7 vorgenommen. Je nach der von der zentralen Steuereinheit 8 über die Dosiereinrichtung 3 gesteuerten momentanen Mengenzufuhr des festen Stoffes, wird entsprechend dem vorgegebenen abgespeicherten Mischungsverhältnisses von der zentralen Steuereinheit 8 auch die Zufuhr der Suspension gesteuert. Die Zuführung der im Vorratsbehälter 4 enthaltenen Suspensionen erfolgt mittels der Dosiereinrichtung 5, die von der zentralen Steuereinheit 8 gesteuert wird. Über die Regelbaugruppe 10 wird die Transportgeschwindigkeit des Mischgutes mittels einer Förderpumpe gesteuert. Vorgegeben wird diese Transportgeschwindigkeit durch entsprechende Steuersignale von der zentralen Steuereinheit 8. Die Transportgeschwindigkeit und Dauer des Transportes richtet sich dabei nach dem erforderlichen Bedarf an Mischgut. Bei einem auftretenden Überdruck in der Transportleitung wird mittels der Regelbaugruppe 10 eine redundante Transportleitung 9 zugeschaltet und ein Alarmsignal erzeugt. Über die Regelbaugruppe 10 und den redundanten Transportleitungen 9 wird das Mischgut in die für die Zwischenlagerung vorgesehenen Behälter 11 geleitet. Zweeks Verhinderung der Schadwirkungen der in Bio- und Zersetzungsgasen vorhandenen, für den Menschen giftigen Konzentration an H₂S- Bestandteilen sowie anderen Gasbestandteilen ist es erforderlich, die austretenden Biogase abzufackeln. Insbesondere beim Auftreten eines Havariefalles innerhalb der Biogasanlage, z. B. durch Ausfall des Motors einer mittels Biogas betriebenen Heizungsanlage, ist ein sofortiges Herunterfahren der Anlage vorgeschrieben. Dies bedeutet die Unterbrechung der Substratzufuhr zu den Biogas erzeugenden Vorrichtungen (z . B. Fermenter) und der Befüllung der Hydrolyse- und Mischbehälter. Die weiterhin bis zur Reduzierung auf die Hälfte der Ausgangsmenge erzeugte Biogasmenge innerhalb eines auf die Havarie folgenden Zeitraumes von 48 Stunden muss abgefackelt werden. Ausgegangen wird bei dem nachfolgend dargestellten Beispiel von einem Ausfall eines Motors innerhalb eines Biogas-Heizkraftwerkes. Bei Ereigniseintritt wird sofort die Substratzufuhr zu den Fermentor-Baugruppen unterbrochen. Dies geschieht durch Abschaltung der Substratpumpe, die aus dem vorgeordneten Hydrolyse- und Mischbehälter das Substrat in die Fermentor-Baugruppen pumpt. Ebenso wird die Befüllung des Hydrolyse- und Mischbehälters unterbrochen. Dazu werden die Pumpen für die Güllezufuhr, für die im Kreislauf geführte Gärrestmenge, für die Wasserzufuhr und der Zufuhr des Getreidebreies abgeschaltet. Mit der Unterbrechung der Substratzufuhr wird eine Reduzierung der erzeugten Biogasmenge innerhalb von 48 Stunden auf unter 50% der Ausgangsmenge bewirkt. Bis zum Ablauf der Zeitspanne wird weiterhin Biogas produziert, das abgeblasen bzw. abgefackelt werden muss. Um die weiterhin erzeugte Biogasmenge weitestgehend in Energie umzuwandeln, wird ein dafür vorgesehener Gaskessel innerhalb eines Heizcontainers zugeschaltet, der z. B. landwirtschaftliche Stallanlagen beheizt. Nach Ablauf der Zeitspanne von 48 Stunden wird die maximal erforderliche Biogasmenge für den Gaskessel erreicht, die gleichzeitig dem 50%-igen Anteil der Ausgangsmenge des Biogases entspricht. Spätestens zu diesem Zeitpunkt ist der Abblasvorgang nicht mehr erforderlich. Nach Ablauf der 48 Stunden wird dann eine vollständige energetische Verwertung des erzeugten Biogases vorgenommen. Sollte die Erzeugung des Biogases auf unter 40% der Ausgangsmenge fallen, würde wieder aus dem Hydrolyse- und Mischbehälter Substratmaterial den Fermentor-Baugruppen zugeführt und so die Biogaserzeugung auf diesem vorgegebenen Niveau gehalten werden. Nachfolgend soll die erfindungsgemäße Vorrichtung an Hand eines Ausführungsbeispiel näher erläutert werden. Mit der Gesamtansicht in Figur 2 wird der schematische Aufbau der Vorrichtung wiedergegeben. Der Silierbehälter 12 weist in seiner Mitte eine um die vertikale Achse rotierende Schneideinrichtung 13 auf, die zur Zerschneidung der vorher zerkleinerten Silierstoffen wie beispielsweise Rüben, Mais und Gras dient. Die Schneidelemente sind an der Schneideinrichtung 13 so angeordnet, dass sie mit ihrer Schneidkante beim Auftreffen auf das zu schneidende Siliergut keine beschädigende Hebelwirkung an ihrer Befestigungsstelle erzeugen. Sie werden deshalb spiralförmig zur Drehrichtung angeordnet und weichen bei Auftreten von großen erforderlichen Schneidkräften zum Mittelpunkt der Schneideinrichtung 13 hin aus. Ein Abbrechen der Schneidelemente wird so vermieden. Um Schäden am Antriebsaggregat der Schneideinrichtung 13, z. B. durch ein Verklemmen der Silierstoffe, zu verhindern, ist zwischen der Antriebsachse und dem Antriebsaggregat eine an sich bekannte Überlasteinrichtung 14 geschaltet. Die für die Vermischung vorgesehene vorgemischte, pastöse dickflüssige Biomasse wird über die Steigleitung 15 zugeführt. In der Steigleitung 15 sind Öffnungen 16 eingebracht aus der die stufenweise Zuführung der Biomasse erfolgt. Die Öffnungen 16 sind höhenversetzt eingebracht und ermöglichen so eine stufenweise Steuerung der Zufuhrmengen. Je nach Füllhöhe der vorher zerkleinerten Silierstoffe und nach Fortschritt des stufenweisen Schneidvorganges werden von außen die jeweiligen Zufuhrmengen gesteuert. Über den im oberen Bereich des Silierbebälters 12 eingebrachten Abführkanal 21 wird der über einen vorgegebenen Gasdruck hinausgehende Anteil an Kohlendioxid aus dem Silierbehälter 12 in einen Vorratsbehälter geleitet und bei der Entstehung eines Unterdruckes zwecks Druckausgleich wieder zurückgeführt. Dies wird mittels einer an sich bekannten Überdruck/ Unterdrucksicherung bewirkt. Auf der Bodenfläche 18 des Silierbehälters 12 sind Erhebungen aufgebracht, die zur Verbesserung bzw. Beschleunigung des Entnahmevorganges der verarbeiteten Silierstoffe dienen. Die Entnahme erfolgt über Entnahmekanäle 20. Um den erforderlichen Druck durch das Eigengewicht der Silierstoffe gezielt auf die Entnahmekanäle 20 zu konzentrieren, weisen die Erhebungen 19 eine nach oben sich verjüngende Form auf. Durch die schrägen Oberflächen der Erhebungen 19 wird das Siliergut in Richtung der Öffnungen der Entnahmekanäle geschoben. Als mögliche Formen der Erhebungen kommen bevorzugt kegelförmige oder pyramidenförmige Gestaltungen in Betracht. Die Wahl der jeweilig Anzahl und der zur Anwendung kommenden Form hängt von der Größe und Form des Silierbehälters 12, der Menge des aufzubereitenden Siliergutes, der Anzahl der Entnahmekanäle 20 und des Herstellungsverfahrens des Silierbehälters 12 ab. So hat beispielsweise eine pyramidenförmige Ausbildung der Erhebung 19 gegenüber einer kegelförmigen bei gleicher Höhe und Schräge eine um ca. 25 % größere Oberfläche. D. h. auf den schrägen Oberflächen könnte eine dementsprechend größere Menge an Silierstoffen den Entnahmekanälen 20 zugeleitet werden. Ein die einzelnen Einflussgrößen berücksichtigendes mathematisches Modell dient als Unterstützung für die Ermittlung der optimalen Gestaltung der Vorrichtung. Unterhalb der Schneideinrichtung 13 ist eine ringförmige aus einem Rohrsystem bestehende Reinigungsvorrichtung 17 angeordnet, die die Oberflächen der Erhebungen 19 im Leerstand des Silierbehälters 12 reinigt. Dazu werden Reinigungsmaterialien von außen zugeführt und auf die Oberflächen gesprüht. Die Oberflächen der Erhebungen 19 weisen zudem eine Nano-Oberflächenbeschichtung mit einer selbstreinigenden Wirkung auf. Mit dem Einsatz dieser Nano-Oberflächenbeschichtung wird eine für die sonst erforderlichen Reinigungsvorgänge entsprechende Energie eingespart. Die Oberflächen erhalten durch diese Beschichtung eine geringere mechanische Reibung gegenüber den auf ihnen gleitenden Silierstoffen. Zwecks Erhaltung einer optimalen Innentemperatur im Bereich von 16° bis 30° C ist an der Bodenfläche 18 des Silierbehälters 12 unterhalb der Oberflächen der Erhebungen 19 eine geregelte Bodenheizung 22 angeordnet. Die Vergrößerung der Oberfläche, auf der das Siliermaterial aufliegt, durch die kegel- bzw. pyramidenförmige Ausbildung der Erhebungen 19 bewirkt neben der geführten Zuführung einer größeren Menge an Siliermaterial zu den Entnahmekanälen 20 auch eine effektivere gleichzeitigen Wärmezufuhr zu einer größeren Menge an Siliermaterial. Insbesondere wird die Wärmezufuhr kurz vor dem Austritt des Silierstoffes aus dem Silierbehälter 12 vorgenommen und so Energieverluste verringert.

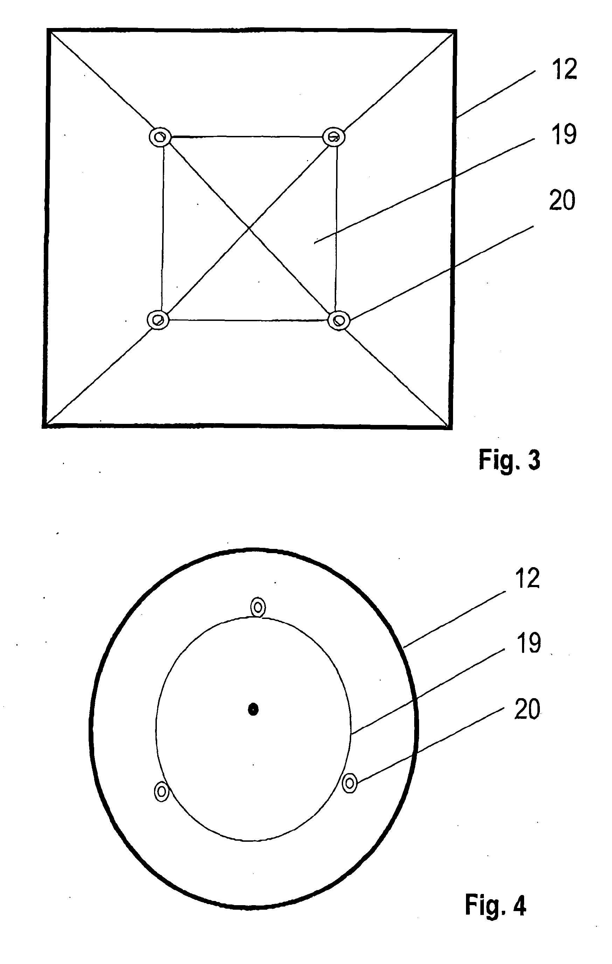

In der Figur 3 ist eine schematische Draufsicht auf die Bodenfläche 18 des Silierbehälters 12 dargestellt. Angeordnet sind bei dieser Darstellung eine pyramidenförmige gestaltete Erhebung 19 und vier an den Ecken der Grundfläche der Pyramide angeordnete Entnahmekanäle 20. Durch die gewählte quadratische Form der Bodenfläche 18 des Silierbehälters 12 stellt eine pyramidenförmige Erhebung 19 die kostengünstigste Variante dar und bietet eine optimale Ausnutzung der Oberflächen. Seitlich um die Erhebung 19 herum sind zu den Wänden des Silierbehälters 12 hin ebenfalls schräge Baugruppen angebracht.

Bei der schematischen Darstellung der Figur 4 wird der Silierbehälter 12 mit einer kreisförmigen Bodenfläche 18 wiedergegeben. In der einfachsten Ausführungsform erweist sich hier die Anwendung einer kegelförmigen Erhebung 19 als vorteilhaft. Neben der vorteilhaften Nutzung der möglichen zu schaffenden schrägen Oberflächen bieten sich günstige Herstellungsmöglichkeiten für die Baugruppen des Silierbehälters 12. Zudem erhält man eine einfache Möglichkeit der Anordnung mehrerer Entnahmekanäle 20. Bezugszeichen
- 1: Schneideinrichtung
- 2: Zwischenspeicher
- 3: Dosiereinrichtung
- 4: Vorratsbehälter
- 5: Dosiereinrichtung
- 6: Mischungsbaugruppc
- 7: Sensor
- 8: zentrale Steuereinheit
- 9: Transportleitung
- 10: Regelbaugruppe
- 11: Behälter
- 12: Silierbehälter
- 13: Schneideinrichtung
- 14: Überlastinrichtung
- 15: Steigleitung
- 16: Öffnung
- 17: Reinigungsvorrichtung
- 18: Bodenfläche
- 19: Erhebung
- 20: Entnahmekanal
- 21: Abführkanal
- 22: Bodenheizung
- 23: Sprühvorrichtung

## Patentansprüche

1. Verfahren zur Energiegewinnung in Biogasanlagen mittels aufbereiteter Mischgüter aus Silierstoffcn durch maschinelle Mischung und danach erfolgender Zwischenlagerung der Mischgüter, **dadurch gekennzeichnet, dass** der Mischvorgang durch technische Regelungen der Zuführungen und Mischungsvorgänge von zerkleinerten, festen pflanzlichen Stoffen mit als Gleitmittel dienenden pflanzlichen Suspensionen erfolgt.

2. Verfahren nach Patentanspruch 1, **dadurch gekennzeichnet, dass** der Füllstand des Mischgutes in der zur Mischung dienenden Mischungsbaugruppe (6) erfasst und als Steuergröße für die Zuführung der Mischungskomponenten dient.

3. Verfahren nach Patentanspruch 1, **dadurch gekennzeichnet, dass** die zugeführte Menge der zerkleinerten, festen pflanzlichen Stoffe während des Zuführungsvorganges pro vorgegebener Zeiteinheit erfasst wird und zwecks Erreichung des jeweilig vorgegebenen Mischungsverhältnisses zur pflanzlichen Suspension die erfassten Werte als Regelungsgröße für die Zuführungsmenge der Suspension dienen.

4. Verfahren nach Patentanspruch 1, **dadurch gekennzeichnet, dass** die zugeführte Menge an pflanzlicher Suspension während des Zufuhrvorganges pro vorgegebener Zeiteinheit erfasst wird und zwecks Erreichung des jeweilig vorgegebenen Mischungsverhältnisses zu den zerkleinerten, festen pflanzlichen Stoffen die erfassten Werte als Regelungsgröße für die Zuführungsmenge der zerkleinerten, festen pflanzlichen Stoffe dienen.

5. Verfahren nach Patentanspruch 1, **dadurch gekennzeichnet, dass** die Strömungsgeschwindigkeit des Mischgutes bei gleichbleibenden Strömungsdruck innerhalb der der Mischungsbaugruppe (6) nachgeordneten Transportleitungen erfasst wird und Abweichungen eines vorgegebenen Wertes über einen Toleranzbereich hinaus signalisiert werden.

6. Verfahren nach Patentanspruch 1, **dadurch gekennzeichnet, dass** zwecks Vermeidung von Rückstau, hervorgerufen durch eine zu hohen Zuführungsmenge an Suspensionen und/oder Verstopfungen der Transportleitungen, mittels einer Regelbaugruppe (10) mindestens eine redundante parallel angeordnete Transportleitung (9) zugeschaltet wird.

7. Verfahren nach Patentanspruch 1, **dadurch gekennzeichnet, dass**
a) das von der Biogasanlage erzeugte Biogas, während eines Havariefalles bei den Energie umwandelnden Baugruppen, mindestens einem Gasspeicher und/oder mindestens einer redundanten, Energie umwandelnden Baugruppe zugeleitet wird und
b) ein Regelung der Zuführung der Biogas erzeugenden Substanzen und der Temperatur der Fermente auf das zur Speicherung des Biogases und/oder zur Energieumwandlung vorgesehene Niveau vorgenommen wird.

8. Verfahren nach Patentanspruch 1, **dadurch gekennzeichnet, dass** mindestens die während des Herunterfahrens erzeugte Biogasmenge dem Gasspeicher und/oder der redundanten, Energie umwandelnden Baugruppe zugeleitet wird.

9. Vorrichtung zur Durchführung des Verfahrens nach Patentanspruch 1 zum kontinuierlichen Verarbeiten von festen Silierstoffen unter Zuführung aufbereiteter dickflüssiger Biomasse durch Zufuhrkanäle und mit einer zur Abführung des erzeugten Kohlendioxides dienenden Einrichtung, **dadurch gekennzeichnet, dass** die Zuführung der aufbereiteten dickflüssigen Biomasse mittels in unterschiedlichen Höhen innerhalb der Vorrichtung angeordneten Zuführkanälen erfolgt, die Bodenfläche (18) der Vorrichtung mindestens eine nach oben hin sich verjüngende Erhebung (19) aufweist, an der Bodenfläche (18) zwischen den Erhebungen (19) mindestens ein Entnahmekanal (20) angeordnet ist und den Oberflächen der Erhebungen (19) eine steuerbare Reinigungsvorrichtung (17) sowie eine Heizeinrichtung funktionell zugeordnet sind.

10. Vorrichtung nach Patentanspruch 9, **dadurch gekennzeichnet, dass** die Erhebung (19) kegelförmig ausgebildet ist.

11. Vorrichtung nach Patentanspruch 9, **dadurch gekennzeichnet, dass** die Erhebung (19) pyramidenförmig ausgebildet ist.

12. Vorrichtung nach Patentanspruch 9, **dadurch gekennzeichnet, dass** die Entnahmekanäle (20) mit einem den Entnahmedruck steuerbaren Pumpsystem funktionell verbunden sind.

13. Vorrichtung nach Patentanspruch 9, **dadurch gekennzeichnet, dass** die Außenwand der Vorrichtung mit einer automatischen Heizeinrichtung verbunden ist, die eine Konstanthaltung der Innentemperatur im Bereich von 16° bis 30° C bewirkt.

14. Vorrichtung nach Patentanspruch 9, **dadurch gekennzeichnet, dass** die Oberflächen der Erhebungen (19) mit einer in der Vorrichtung angeordneten automatischen Bodenheizung (22) funktionell verbunden sind.

15. Vorrichtung nach Patentanspruch 9, **dadurch gekennzeichnet, dass** die Zufuhrkanäle als in einer Steigleitung (15) höhenversetzt angeordnete Öffnungen (16) ausgebildet sind.

16. Vorrichtung nach Patentanspruch 9, **dadurch gekennzeichnet, dass** im oberen Bereich der Vorrichtung ein Abführkanal (21) angeordnet ist, über den bei der Erreichung eines vorgegebenen Gasdruckes mittels einer Regeleinrichtung die Ableitung von Kohlendioxid in einen Aufnahmebehälter bewirkt wird.

17. Vorrichtung nach Patentanspruch 9, **dadurch gekennzeichnet, dass** innerhalb der Vorrichtung eine um die vertikale Mittelachse der Vorrichtung rotierende Schneideinrichtung (13) angebracht ist, die mit Schnittelementen versehen ist, die die Schnittvorgange an den festen Silierstoffen spiralförmig zur Drehbewegung ausführen.

18. Vorrichtung nach Patentanspruch 9, **dadurch gekennzeichnet, dass** im oberen Bereich der Vorrichtung eine zur Beschleunigung des Siliervorganges und/oder Konservierung dienende und Konservierungsmittel zuführende steuerbare Sprühvorrichtung (23) angeordnet ist
